# EUROPEAN PATENT APPLICATION

(11) **EP 4 702 934 A1**
(43) Date of publication of application: **04.03.2026**
(21) Application number: 24196384.2
(22) Date of filing: 26.08.2024
(51) Int. Cl.: A61B 8/08

(54) **PROCESSING ULTRASOUND IMAGE DATA**

(71) Applicant: Koninklijke Philips N.V., 5656 AG Eindhoven (NL)
(72) Inventor: AIRSANG, Urmila, 5656 AG Eindhoven (NL); FIRTION, Celine, 5656 AG Eindhoven (NL); NANJUNDEGOWDA, Navya, 5656 AG Eindhoven (NL); ATCHYUT, Akella Sivasai, 5656 AG Eindhoven (NL); VELUMURGAN, Mylavan, 5656 AG Eindhoven (NL); SUBASH CHANDRAN, Dheepak, 5656 AG Eindhoven (NL); AWASTHI, Manish, 5656 AG Eindhoven (NL); SETH, Subhendu, 5656 AG Eindhoven (NL); BRAHMA, Deb Kumar, 5656 AG Èindhoven (NL); VAJINEPALLI, Pallavi, 5656 AG Eindhoven (NL)
(74) Representative: Philips Intellectual Property & Standards

(57) **Abstract**

A mechanism for identifying the orientation of a representation of a fetus within ultrasound image data. The ultrasound image data is formed from a sequence or stack of image slices. The ultrasound image data is processed to identify image slices that provide different predetermined views of the fetus. The order in which the identified image slices appear in the sequence or stack of image slices is processed to identify the orientation of the fetus.

## Description

### FIELD OF THE INVENTION

The present disclosure relates to the field of medical imaging, and in particular, to ultrasound imaging.

### BACKGROUND OF THE INVENTION

There is an increasing interest in and reliance upon medical imaging procedures within the medical profession. One particular area in which medical imaging, particularly ultrasound imaging, has proven of vital importance is obstetrics for monitoring and assessing a growing fetus.

Ultrasound imaging benefits from the capability of being non-invasive during typical use, as well as facilitating the imaging of most of the internal parts of the body (e.g., like bones, tissue etc.). One analytic technique that may be performed during ultrasound imaging of a fetus is to analyze, assess and/or detect anomalies within the fetus, such as heterotaxy syndrome/ isomerism. This later abnormality is a result of abnormal symmetry of the viscera that are normally dissimilar due to abnormal lateralization of thoracic and abdominal viscera.

There is an ongoing desire to process ultrasound information to produce additional data or indicators that would aid a clinician or operator in analyzing any ultrasound images.

### SUMMARY OF THE INVENTION

The invention is defined by the claims.

According to examples in accordance with an aspect of the invention, there is provided a computer-implemented method for processing ultrasound image data of a fetus.

The computer-implemented method comprises: receiving ultrasound image data containing a 3D representation of a portion of the fetus, wherein the ultrasound image data is formed from a temporal sequence of image slices, each image slice being captured at a different point in time; processing the ultrasound image data to identify, for each of a plurality of predetermined views of the fetus, a respective image slice containing a representation of said predetermined view; and predicting an orientation of the representation of the portion of the fetus within the ultrasound image data responsive to an order in which the identified image slices are located within the temporal sequence of image slices.

The present disclosure provides a new mechanism for identifying the orientation of a (portion of a) fetus represented with ultrasound image data. The ultrasound image data contains a 3D representation of a portion of the fetus. The ultrasound image data is conceptually formed from a stack of image slices, which effectively form a sequence of image slices. Each image slice is derived from a respective instance of imaging data that is captured at/over a different point/period of time. This is a natural consequence of an ultrasound imaging procedure in which a sweep of a subject's anatomy is performed. In this way, the sequence of image slices is a temporal sequence of image slices, in which slices later in the sequence are derived from imaging data captured at a later point in time than slices earlier in the sequence.

It has been recognized that the order in which predetermined views, provided by the image slices, appear in the stack of image slices indicate an orientation of the (portion of the) fetus with respect to the stack of image slices, and therefore the ultrasound image data. Thus, by identifying image slices that represent a set of predetermined views, it is possible to identify an order in which the views appear within the sequence of image slices. This order defines (e.g., can be mapped to) an orientation of the (portion of the) fetus.

A determined orientation of a fetus within ultrasound image data provides or defines valuable technical information about the fetus, which may, for instance, be advantageously used by a clinician (in their assessment of the fetus) and/or further processing steps. This may, for instance, be particularly useful in medical scenarios where understanding the precise orientation of the fetus is of paramount concern, such as when planning a surgical procedure or when monitoring for specific fetal conditions.

More particularly, orientation of the fetus can provide insights into the positioning of the fetus within the womb (e.g., indicating a progress towards birth), the alignment of various anatomical structures, and potential abnormalities in fetal development.

In some examples, the step of predicting the orientation comprises: determining an order in which the identified image slices appear in the temporal sequence of image slices; and mapping the determined order to an orientation of the representation of the portion of the fetus. The mapping may be performed using one or more predetermined rules and/or a mapping table, e.g., which maps or defines a relationship between a plurality of different orders and respective orientations of the representation of the portion of the fetus.

This provides a simple and reliable mechanism for identifying the orientation of the (portion of the) fetus within the ultrasound image data, as it exploits the recognition that the order of the predetermined views will map or relate to the orientation of the (portion of the) fetus.

Preferably, the orientation of the representation of the portion of the fetus represents an overall orientation of the fetus. In other words, the orientation of the (portion of the fetus) may align with the overall orientation of the fetus. Put another way, the orientation of the portion of the fetus may be effectively defined by the orientation of the overall fetus (e.g., a head-toe orientation or a head-bottom orientation).

In some examples, the plurality of predetermined views includes at least one view of the heart of the fetus. In some examples, the plurality of predetermined views includes two or more different views of the heart of the fetus.

The computer-implemented method may further comprise processing the ultrasound image data to identify, as a spine location, a location of a representation of the spine of the fetus in the ultrasound image data; and processing the identified spine location and the predicted orientation of the representation of the portion of the fetus to identify a left side and a right side of the fetus in the ultrasound image data.

In this way, a laterality of the representation of the fetus in the ultrasound image data can be automatically detected using the identified spine location. This embodiment is based on the realization that a side of the fetus can be identified based on knowledge of the spinal location and the orientation of the fetus.

In some examples, the method comprises for each of at least one target image slice of the ultrasound image data, processing the target image slice to identify a location of a bisecting line that is predicted to bisect the target image slice into two portions, each portion representing a different half of the representation of the portion of the fetus; and generating identifying information, the identifying information identifying which portion of each target image slice represents the left side of the fetus and which portion represents the right side of the fetus, using the identified spine location and the predicted orientation of the representation of the portion of the fetus.

The computer-implemented method may further comprise, for each target image slice, annotating the portions of the target image slice using the identifying information to indicate which portion represents the right side and which portion represents the left side.

In some examples, the computer-implemented method comprises further comprising, for each target image slice, processing the target image slice to identify the bounds of the representation of the fetus within the target image slice, wherein annotating the portions of the target image slice comprises annotating the target image slice outside the identified bounds of the representation of the fetus. This reduces a risk that the annotation(s) will overlay or otherwise obscure or block the representation of the fetus. This reduces a risk that potentially relevant or important detail of the condition of the fetus will be overlooked, e.g., during any subsequent display or analysis of the ultrasound image data.

The computer-implemented method may further comprise controlling a display to provide a visual representation of at least one target image slice including the annotations of the left side and the right side of the fetus.

The computer-implemented method may further comprise processing the identifying information using a situs detection algorithm to identify the position of each representation of one or more anatomical elements and/or anatomical organs within one or more of the at least one target image slices.

The computer-implemented method may further comprise controlling a display to provide a visual representation of the ultrasound image data and a visual representation of the predicted orientation.

There is also provided a computer program product comprising computer program code means which, when executed on a computing device having a processing system, cause the processing system to perform all of the steps of any herein disclosed method.

There is also provided a processing system for processing ultrasound image data of a fetus. The processing system is configured to: receive ultrasound image data containing a 3D representation of a portion of the fetus, wherein the ultrasound image data is formed from a temporal sequence of image slices, each image slice being captured at a different point in time; process the ultrasound image data to identify, for each of a plurality of predetermined views of the fetus, a respective image slice containing a representation of said predetermined view; and predict an orientation of the representation of the portion of the fetus within the ultrasound image data responsive to an order in which the identified image slices are located within the temporal sequence of image slices.

The processing system may be adapted to perform the steps or functions of any herein disclosed method and vice versa.

There is also provided a system comprising: an ultrasound imaging system configured to generate the ultrasound image data; and the processing system. Of course, the system may comprise one or more other elements, such as a database or memory for storing the ultrasound image data and/or a determined orientation; a user interface (e.g., providing a display) and so on.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a system in which embodiments may be employed;
Fig. 2 illustrates a proposed method;
Fig. 3 illustrates an approach to obtaining ultrasound image data;
Fig. 4 illustrates a further proposed method;
Fig. 5 illustrates a further proposed method;
Fig. 6 illustrates an image slice for use in a proposed method;
Fig. 7 illustrates an approach to identifying laterality; and
Fig. 8 illustrates a proposed processing system.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for identifying the orientation of a representation of a fetus within ultrasound image data. The ultrasound image data is formed from a sequence or stack of image slices. The ultrasound image data is processed to identify image slices that provide different predetermined views of the fetus. The order in which the identified image slices appear in the sequence or stack of image slices is processed to identify the orientation of the fetus.

Embodiments are based on the realization that the order in which data representing certain views are obtained during an ultrasound imaging procedure indicate the orientation of a fetus with respect to the overall imaging data produced by the ultrasound imaging procedure. The identified order can therefore be exploited or leveraged to identify the orientation.

In the context of the present disclosure, a view of an image (slice) is defined by the anatomical elements and/or viewpoint location and/or viewing angle provided by the image (slice). In some examples, a view of an image (slice) may be considered to define a label that defines the content and viewing angle provided by the image (slice). A wide variety of views for ultrasound images of a fetus are known in the art, such as those set out in the Fetal Anomaly Screen Programme Handbook, NHS Screening Programmes, London, U.K., 2015.

In the context of the present disclosure, an orientation of a fetus is considered to be a head-toe orientation or a head-bottom orientation. The precise definition of an orientation of the fetus may, for instance, depend upon the precise environment in which the proposed approach is employed.

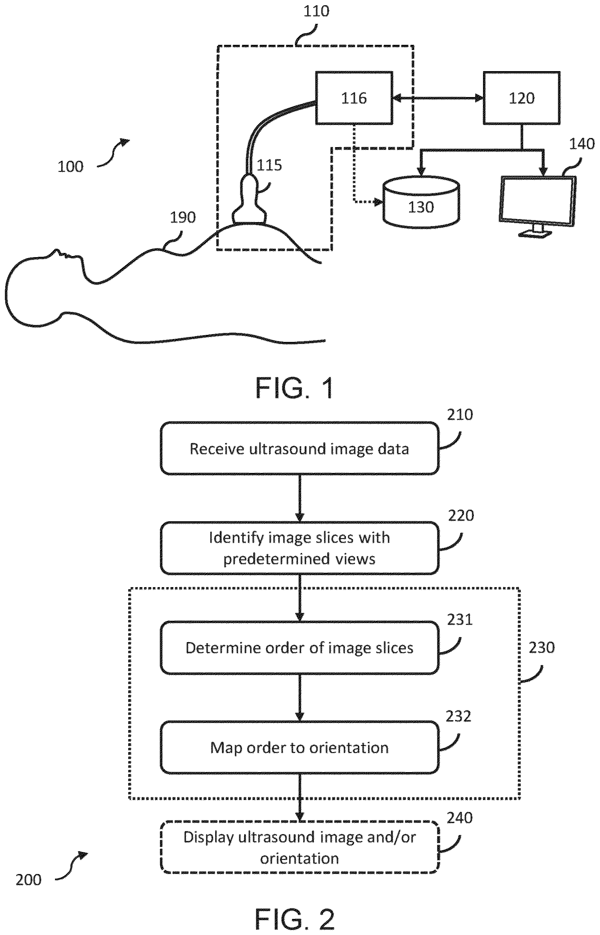

Fig. 1 illustrates a system 100 in which proposed embodiments may be employed, for improved contextual understanding. The system 100 here comprises an ultrasound imaging system 110, a processing system 120, a database 130 and a user interface system 140.

The ultrasound imaging system 110 is configured to obtain, collect, or otherwise acquire 3D ultrasound imaging data of a subject 190. 3D ultrasound imaging data is data that forms, or may otherwise be reconstructed into ultrasound image data that represents a 3D volume comprising a portion of the subject's body. In other words, the ultrasound image data comprises a 3D representation of a portion of the subject's body. The reconstruction may be performed by the ultrasound imaging system 110 itself or the processing system 120.

The ultrasound image data is formed from a series of slices/images (e.g., 2D slices or 3D slices) that correspond to a set of one or more layers of a 3D dataset, i.e., discrete datasets "stacked" along a particular axis of the ultrasound image data. Each slice therefore represents a slice/cut through the volume represented by the ultrasound image data, e.g., a slice through the subject.

The general function of an ultrasound imaging data is well known in the art, and is not described in detail for the sake of conciseness.

Nonetheless, it is noted that in a typical ultrasound imaging system capture of the 3D imaging data is performed by sequentially capturing respective imaging data for each image slice of the ultrasound image data using an ultrasound probe 115 (e.g., in communication with an imaging system processor 116). The position of the ultrasound probe 115 can be moved between each sequential capture of image data, such that each instance of imaging data represents a different part or portion of the imaged 3D volume.

Typically, the ultrasound probe 115 has at least one transducer array having a multitude of transducer elements for transmitting and/or receiving ultrasound waves. In one example, each of the transducer elements can transmit ultrasound waves in form of at least one transmit impulse of a specific pulse duration, in particular a plurality of subsequent transmit pulses.

Thus, due to the nature of image capture by the ultrasound imaging system, each image slice is captured at or over a different point/period of time. In this way, the ultrasound image data is formed from a temporal sequence of image slices, each image slice being captured at a different point in time. Each slice may, for instance, represent a single layer of the 3D dataset or, alternatively, a stack of two or more layers of the 3D dataset.

In this way, the ultrasound image data is formed from a (temporal) sequence of image slices, in which image slices later in the sequence are derived from imaging data captured later in time than image slices earlier in the sequence.

For the avoidance of doubt, it is noted that each image slice may be a 2D or a 3D slice of the (larger) ultrasound image data.

It will be appreciated that the ultrasound image data represents a 3D volume (of interest) containing a portion of the fetus. The ultrasound image data may comprise, for instance, a 3D dataset having a discrete number of regularly arranged (e.g., in rows, columns and layers) data points, each data point being a data value (i.e., a numerical value) that is associated with a position (e.g., an (x, y, z) coordinate) in the 3D volume. In most cases, the data values are intensity values that indicate one or more properties/characteristics of the materials/substances comprised within the 3D volume (e.g., air, bone, soft tissue, etc.).

Each data point of the ultrasound image data may effectively represent a pixel/voxel when displayed in the form of a visual representation. In particular, the data value of each data point can be mapped to a corresponding intensity/brightness/color for the voxels/pixels, according to appropriate color scales and/or contrast settings. In this way, each data point effectively represents a different sub-volume of the 3D volume represented by the ultrasound image data.

The ultrasound imaging system 110 may be configured to provide the 3D ultrasound imaging data and/or the ultrasound image data (if produced by the ultrasound imaging system) to the database 130. More specifically, the ultrasound imaging system 110 may store the medical imaging data and/or the ultrasound image data (if available) in the database 130. Once stored in the database 130, the medical imaging data and/or the 3D image may be retrieved (e.g., at a later time) by the processing system 120 and/or the user interface system 140.

In particular, the database 130 may be a PACS database that allows for the confidential storage and retrieval of medical images and imaging data between different medical devices and different medical institutions.

In some examples, the ultrasound imaging system 110 and/or the processing system 120 may be configured to control the user interface 140 to provide a visual representation of (i.e., display) the ultrasound image data or a portion thereof (e.g., a particular slice or part of a slice of the ultrasound image data). This visual representation may be provided via a screen of the user interface system 140. The user interface system 140 may comprise one or more input peripherals for receiving a user input to control the display of the ultrasound image data, e.g., to rotate the ultrasound image data and/or select which portion(s) of the ultrasound image data is displayed (e.g., which slice of the ultrasound image data is displayed).

It will be appreciated that when the ultrasound imaging system is used to image a fetus, then the ultrasound image data contains a representation of at least a portion (e.g., some or all) of the fetus.

The present disclosure provides a mechanism for predicting the orientation of a representation of a portion of a fetus within ultrasound image data, such as that produced using the above described ultrasound imaging system 110. More specifically, slices of the 3D image(s) represented known/predetermined views of the fetus are identified. The order in which these slices with predetermined views appear in the ultrasound image data indicate an orientation of the fetus.

By way of simple example, if a slice containing a view of the heart of the fetus is temporally before a slice containing a view of the abdomen of the fetus, then it can be assumed that the fetus is generally orientated in a direction that points from later slices in the sequence to earlier slices in the sequence.

Fig. 2 is a flowchart illustrating a proposed computer-implemented method 200 for processing ultrasound image data of a fetus. The method 200 may be performed by the processing system previously mentioned, a more detailed example of which will be provided later in this disclosure.

The method 200 comprises a step 210 of receiving ultrasound image data containing a representation of a portion of the fetus. As previously explained, the ultrasound image data is formed from a temporal sequence of image slices, each image slice being captured at a different point in time (e.g., over a different window/period of time).

Step 210 may be performed, for instance, by directly retrieving or receiving the ultrasound image data from an ultrasound imaging system and/or database. In other examples, step 210 may be performed by performing a reconstruction process using 3D ultrasound imaging data produced by the ultrasound imaging system. Examples of such approaches will be well known to the skilled person.

The method 200 also comprises a step 220 of processing the ultrasound image data to identify, for each of a plurality of predetermined views of the fetus, a respective image slice containing a representation of said predetermined view.

Examples of predetermined views of the fetus that may be employed for the purposes of step 220 include: an abdomen view; an anteroposterior femur view; a sagittal spine view; a coronal spine view; a profile view (e.g., of the face); a four chamber (4CH) view of the heart; a three vessel view (3VV) of the heart; a right ventricular outflow tract (RVOT) view of the heart; a left ventricular output tract (LVOT) view of the heart; a three vessels and trachea view (3VT). Other examples of predetermined views for a fetus will be readily apparent to the skilled person, e.g., upon consulting fetal assessment guidelines or the like.

In some examples, the plurality of predetermined views includes at least one view of the heart of the fetus. More preferably, the plurality of predetermined views includes two or more different views of the heart of the fetus.

A wide variety of approaches for performing step 220 are envisaged for use in the present disclosure.

By way of example only, the European Patent Applications having publication numbers EP 4,041,086 A1 and EP 4,091,550 A1 disclose approaches for identifying a view provided by the ultrasound image. Any one or more of these approaches may be employed to identify or determine a view provided by the image slice(s).

In one working example, step 220 may comprise processing each image slice of the ultrasound image data using one or more classifications algorithms to classify, and therefore identify, a view provided by the image slice. For instance, each predetermined view may be associated with a different classification algorithm trained to predict whether or not an image slice contains a representation of the associated predetermined view. Each image slice may be processed with each classification algorithm to identify those image slices associated with the predetermined views.

Approaches for processing an ultrasound image to identify a view (e.g., an anatomical view plane) of the ultrasound image are well known in the art, such as those disclosed by Kumar, Ashnil, et al. "Plane identification in fetal ultrasound images using saliency maps and convolutional neural networks." 2016 IEEE 13th International Symposium on Biomedical Imaging (ISBI). IEEE, 2016; and/or Baumgartner, Christian F., et al. "SonoNet: real-time detection and localisation of fetal standard scan planes in freehand ultrasound." IEEE transactions on medical imaging 36.11 (2017): 2204-2215.

In another working example, step 220 may comprise processing each image slice using one or more object detection algorithms. For any image slice for which at least one object is detected, the detected object(s) are mapped to a corresponding view for the image slice. For instance, if it is detected that an image slice contains a representation of a portion of the brain, then the view may be determined to be a view of the head or brain of the fetus.

Examples of suitable object detection algorithms are also well known in the art, such as you only look once (YOLO) algorithm first proposed by Redmon, Joseph, et al. "You only look once: Unified, real-time object detection." Proceedings of the IEEE conference on computer vision and pattern recognition. 2016. Other examples will be readily apparent to the skilled person.

A wide variety of other techniques will be readily apparent to the appropriately skilled person.

In some examples, step 220 further comprises performing a data cleaning procedure. For instance, step 220 may comprise discarding any identified image slice which does not form part of a sequence of more than a predetermined number of identified image slices identifying a same predetermined view.

Put another way, step 220 may comprise identifying from the identified image slices (i.e., image slices identified to provide one of the predetermined views) sequences of more than a predetermined number of identified image slices, wherein each identified image slice in a same sequence provides a same predetermined view. Other identified image slices may be discarded or ignored for the purposes of the remainder of the method 200.

The predetermined number may, for instance, be no less than 4, e.g., no less than 10. The predetermined number may be a tunable parameter, e.g., user selectable.

This approach helps to reduce a risk of noise, inaccuracies/error in view classification and/or random variation in the imaging procedure (e.g., a rogue movement of the ultrasound probe) from affecting the proposed method. Thus, the proposed method is made more robust.

The method 200 also comprises a step 230 of predicting an orientation of the representation of the portion of the fetus within the ultrasound image data responsive to an order in which the identified image slices are located within the temporal sequence of image slices.

The ultrasound image data is formed from a temporal sequence of slices, which conceptually form a stack of slices. It is therefore possible to define, within the ultrasound image data, a first direction that extends from a first or an earlier slice in the sequence of slices to the last or a later slice in the sequence and a second direction (opposite to the first direction) that extends from the last or the later slice in the sequence to the first or the earlier slice in the sequence.

Embodiments are at least partially based on the realization that the order in which certain views or anatomical objects appear in the sequence of image slices (forming the ultrasound image data) effectively indicate in which direction the representation of the fetus is lying within the ultrasound image data.

In some embodiments, if the temporal sequence of slices contains two or more views in the order of abdomen, 4CH, LVOT, RVOT, 3VV and then 3VT, then it can be assumed that the fetus lies generally in the first direction (i.e., the head of the fetus is angled towards the last or the later slice in the sequence, rather than the first slice or the earlier slice). If the temporal sequence of slices contains two or more views in the order of 3VT, 3VV, RVOT, LVOT, 4CH, and then abdomen, then it can be assumed that the fetus lies generally in the second direction (i.e., the head of the fetus is angled towards the first or the earlier slice in the sequence, rather than the last slice or the later slice).

For instance, if a slice representing a portion of the heart of the fetus is earlier in the sequence of slices than a slice representing a portion of the head of the fetus or later than a slice representing a portion of the abdomen of the fetus, then it can be assumed that the fetus lies generally in the first direction.

As another example, if a slice representing a 4-chamber (4CH) view of the heart of the fetus is earlier in the sequence of slices than a slice representing a left ventricular outflow tract (LVOT) view of the heart of the fetus, then it can be assumed that the fetus lies generally in the first direction).

As yet another example, if a slice representing a LVOT view of the heart of the fetus is sequentially earlier than a slice representing a RVOT view of the heart which, in turn, is sequentially before a slice representing a 3VV of the heart, then it can be assumed that the fetus lies generally in the first direction.

As yet another example, if a slice representing a 3VV view of the heart is sequentially earlier than a slice representing a LVOT view, then it can be assumed that the fetus lies generally in the second direction.

Once the skilled person has understood that the order of the view of particular slices in the ultrasound image data defines or is responsive to the orientation of the fetus, then they would readily and immediately identify a wide variety of other suitable views and corresponding orders that map to particular orientations.

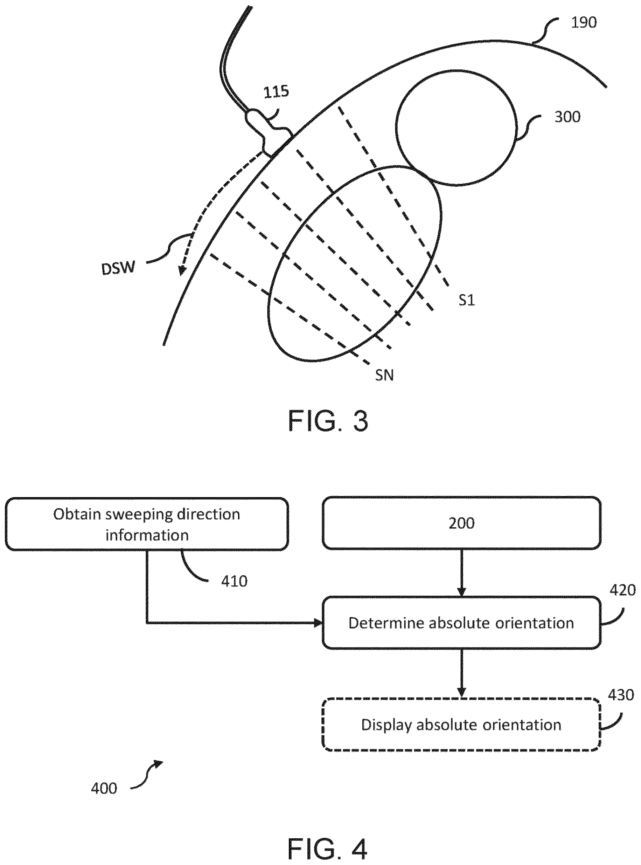

Fig. 3 illustrates, for improved contextual understanding, a representation of a mechanism for obtaining ultrasound image data.

In particular, Fig. 3 illustrates how an ultrasound transducer 115 may be moved over the surface of an individual 190 in a sweeping direction DSW. This movement (in the sweeping direction) is typically controlled or defined by an operator of the ultrasound imaging system, i.e., is a freehand direction. As the ultrasound transducer is moved over the surface of the individual, it is used to iteratively capture instances of ultrasound imaging data. Each instance of ultrasound imaging data is used to derive a respective slice S 1 ... SN of the ultrasound image data. In this way, a sequence of image slices is built up with sequential capture of instances of ultrasound imaging data.

From Fig. 3, it will be clear that the first slice S1 of the ultrasound image data may be derived from data captured at a start of the movement in the sweeping direction DSW. Similarly, the final slice SN of the ultrasound image data may be derived from data captured at an end of the movement in the sweeping direction. Since the sweeping is typically freehand, the sweeping trajectory can be of any form, such as straight, curving, meandering, winding and so on. A direction extending from an earlier slice toward a later slice is often called the forward direction of the sweeping, or simply the sweeping direction. Similarly, a direction extending from a later slice toward an earlier slide is called the backward direction.

For instance, where the fetus 300 lies generally in the "first direction" identified above, it can be considered that the fetus will be generally aligned with the sweeping direction (i.e., pointing in a same direction as the sweeping direction), or in other words, the sweeping moves away the fetus's toe or moves along toe-head direction. Conversely, where the fetus lies generally in the "second direction" identified above, it can be considered that the fetus will be opposite to the sweeping direction, i.e., points towards a position at which the movement in the sweeping direction originated, or in other words, the sweeping moves toward the fetus's toe, as illustrated in Fig. 3.

For the purposes of this disclosure, it is assumed that, for each image slice of the image data, a left side of the image slice is dervied from data captured by a left side of the probe from the perspective of the sweeping directon DSW and that a right side of the image slice is dervied from data captured by a right side of the probe from the perspective of the sweeping directon DSW.

Turning back to Fig. 2, as illustrated therein, step 230 may be performed by determining 231 an order in which the identified image slices appear in the temporal sequence of image slices (i.e., an order in which the predetermined views appear in the sequence); and mapping 232 the determined order to an orientation of the representation of the portion of the fetus. Thus, the order (or sequence) of views in the sequence of image slices effectively determines or defines the orientation of the representation of the portion of the fetus.

In this way, a dataset may store (e.g., in tabular form) information that defines a mapping or relationship between an order of views and the orientation of the fetus in the ultrasound image data.

The method 200 may, in some examples, further comprise a step 240 of controlling a display (e.g., of the user interface) to provide a visual representation of the ultrasound image data and a visual representation of the predicted orientation. The visual representation of the predicted orientation may, for instance, comprise an arrow pointing in the predicted orientation of the fetus.

Approaches for controlling a display to provide a visual representation of an element, such as ultrasound image data and/or information about a predicted orientation, are well known and established in the art.

Fig. 4 is a flowchart illustrating a computer-implemented method 400 that includes further optional steps that may be performed. The method 400 is designed to identify an absolute orientation of the fetus. In this context, an absolute orientation of the fetus is an orientation of the fetus with respect to the pregnant individual. This differs from the orientation of the fetus identified in method 200, which relates to an orientation with respect to (i.e., within) the ultrasound image data (i.e., a "relative orientation").

The method 400 comprises performing the method 200 previously disclosed, to determine an orientation of the fetus in the ultrasound image data.

The method 400 also comprises a step 410 of obtaining sweeping direction information. The sweeping direction information indicates a (general/average) direction that an ultrasound transducer is moved during an ultrasound imaging system, e.g., towards the head of the pregnant individual or away from the head of the pregnant individual.

The sweeping direction information thereby represents a relationship between an orientation of the ultrasound image data and the orientation of the pregnant individual. More particularly, the sweeping direction information will indicate whether the first slice (of the sequence of image slices of the ultrasound image data) is closer to the head of the pregnant individual or whether the last/final slice is closer to the head of the pregnant individual.

The sweeping direction information may be obtained, in step 410, from a user input provided at a user input interface. Thus, an operator may simply input or indicate the sweeping direction to thereby produce the sweeping direction information.

Other approaches for determining sweeping direction information (e.g., through image analysis of the ultrasound imaging process captured by a camera or the like) would readily be apparent to the skilled person.

The method 400 also comprises a step 420 of determining the absolute orientation of the fetus using the sweeping direction information and the orientation of the fetus in the image data. As previously explained, the sweeping direction information represents a relationship between an orientation of the ultrasound image data and the orientation of the pregnant individual.

Thus, it is trivial to determine an absolute orientation of the fetus using the sweeping direction information and the determined orientation.

For instance, if the sweeping direction information indicates that the first slice of the ultrasound image data is closer to the head of the pregnant individual than the last slice, and the orientation indicates that the fetus is directed towards the last slice, then the absolute orientation information will indicate that the fetus is pointed away from the head of the pregnant individual.

As another example, if the sweeping direction information indicates that the first slice of the ultrasound image data is closer to the head of the pregnant individual than the last slice, and the orientation indicates that the fetus is directed towards the first slice, then the absolute orientation information will indicate that the fetus is pointed towards the head of the pregnant individual.

As another example, if the sweeping direction information indicates that the first slice of the ultrasound image data is further from the head of the pregnant individual than the last slice, and the orientation indicates that the fetus is directed towards the last slice, then the absolute orientation information will indicate that the fetus is pointed towards the head of the pregnant individual.

As yet another example, if the sweeping direction information indicates that the first slice of the ultrasound image data is further from the head of the pregnant individual than the last slice, and the orientation indicates that the fetus is directed towards the first slice, then the absolute orientation information will indicate that the fetus is pointed away from the head of the pregnant individual.

The method 400 may, in some examples, further comprise a step 430 of controlling a display (e.g., of the user interface) to provide a visual representation of the absolute orientation. The visual representation of the predicted orientation may, for instance, comprise an arrow indicating the (predicted) absolute orientation of the fetus or a textual representation of the predicted absolute orientation.

Approaches for controlling a display to provide a visual representation of an element, such as an absolute orientation, are well known and established in the art.

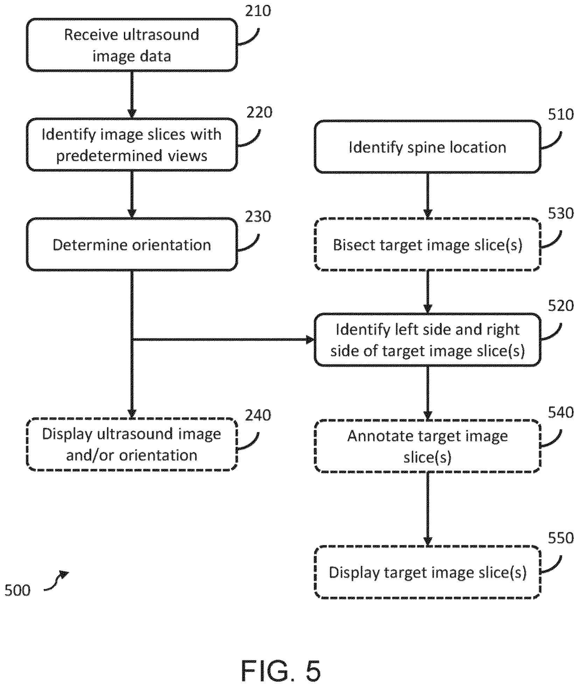

Fig. 5 is a flowchart illustrating a computer-implemented method 500 that includes further optional steps that may be performed. The computer-implemented method 500 may, for instance, be carried out by the processing system.

The computer-implemented method comprises the previously disclosed steps 210, 220 and 230 and optionally step 240. These may be embodied as previously described.

The computer-implemented method 500 further comprises a step 510 of processing the ultrasound image data to identify, as a spine location, a location of a representation of the spine of the fetus in the ultrasound image data.

Step 510 may be performed using an appropriately trained or configured object detection algorithm such as a segmentation algorithm. One appropriate algorithm is an appropriately trained YOLO algorithm, previously mentioned, although other suitable machine-learning algorithms could be used.

The computer-implemented method further comprises a step 520 of processing the identified spine location and the predicted orientation of the representation of the portion of the fetus to identify, for each of at least one target image slices of the ultrasound image data, a representation of the left side and a representation of the right side of the portion of the fetus in the target image slice.

In particular, it has been recognized that the relative position of the spine in the ultrasound image data, together with the determined orientation of the fetus, can be processed to accurately identify the left and right side of the fetus in the ultrasound image data, i.e., which portion of the ultrasound image data represents a left side and which represents a right side.

To facilitate the performance of step 520, in some examples method 500 comprises a further step 530 of, for at least one target image slice of the ultrasound image data, processing at least the target image slice to identify the location of a bisecting line or bisecting surface (e.g., a bisecting plane) that is predicted to bisect the target image slice into two portions, each portion representing a different half of the portion of the fetus. Thus, the bisecting line or bisecting surface is effectively a sagittal line or sagittal surface/plane.

Step 530 may be performed by processing the entire ultrasound image data to identify a bisecting surface that is predicted to bisect the entire ultrasound image data into two portions (representing a different half of the portion of the fetus). Alternatively, step 530 may be performed by processing each image slice individually.

In particular, step 530 may comprise processing the ultrasound image data, or each image slice thereof, to identify a sagittal plane or sagittal line the ultrasound image data or each image slice thereof.

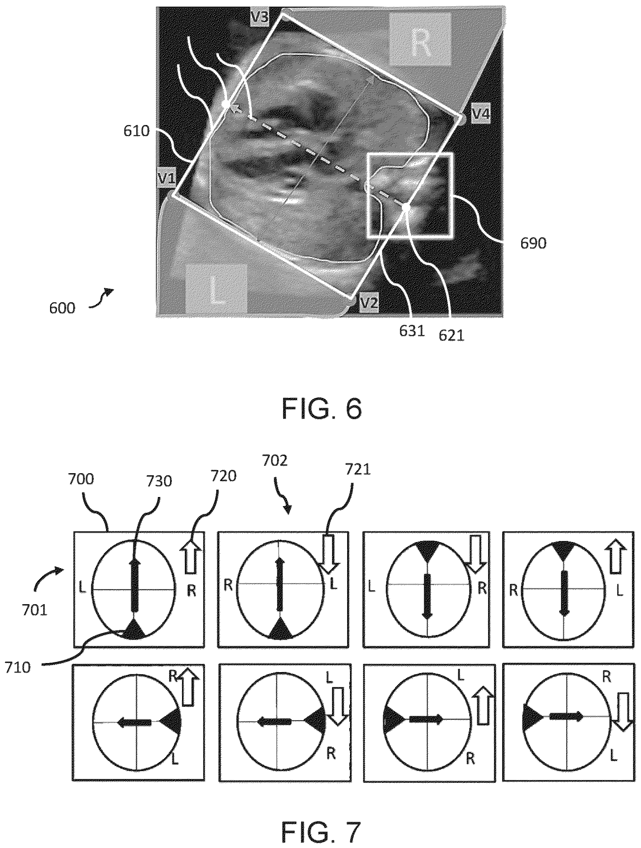

One approach to performing step 530 on a single target image slice is hereafter described with reference to Fig. 6 for the sake of contextual understanding. In this approach, it is assumed that the target image slice is a 2D ultrasound image. Fig. 6 illustrates a target image slice 600 containing a representation of a portion (here: a cardiac portion) of the fetus. The location of the spine has been previously identified (i.e., in a previous step).

In this approach, the target image slice is processed to identify the bounds 610 of the representation of the fetus within the target image slice. As illustrated, the identified bounds 610 may take the form of a bounding box having edges that intersect with the edges 620 of the representation of the fetus. A sagittal line is a line that connects between two sides of the bounding box, including a side most proximate to the identified spine location in the target image slice.

More particularly, the sagittal line for the target image slice may be identified as connecting a first position 621 on a first side 631, being a side of the bounding box 610 most proximate to the identified spine location 690, to a second position 622 on a second side 632 being a side most distant from the identified spine location. The first side 631 is parallel to the second side 62. The first position is a location on the first side that is most proximate to the identified spine location. The second position is a position on the second side that is most proximate to the identified spine location. In this way, the identified sagittal line runs perpendicularly to the first side and the second side.

The portion of the image slice that lies on one side of the identified sagittal line is considered to represent one side of the portion of the fetus, with the portion of the image slice that on another side of the identified sagittal line is considered to represent the other side of the portion of the fetus.

It is also possible to define a forward direction 650 for the target image slice. A forward direction is a direction that extends from the first position to the second position.

Turning back to Fig. 5, step 520 may comprise generating identifying information that identifies which portion of each target image slice represents the left side of the fetus and which portion represents the right side of the fetus. As previously explained, this is performed using the identified spine location and the predicted orientation of the representation of the portion of the fetus.

Fig. 7 illustrates possible target slices for improved contextual understanding, in which the location of the representation of the spine 710 and/or the predicted orientation 720, 721 of the representation of the fetus differs for each target slice.

It is also assumed that the operator orients the ultrasound probe in a standard way during the acquisition of the temporal sequence of the slices. Typically, an ultrasound probe has an orientation marker on one side of the probe (called a probe orientation marker), which can be a ridge, indentation, groove or nub. It corresponds to an orientation marker on the ultrasound image (called image orientation marker). For almost all standard applications and procedures the indicator orientation marker position will be on the left side of the image.

For the purposes of this approach, it is therefore assumed that the left side of the image data represents data captured by the side of the probe that is to the left of a sweeping direction (i.e., the first direction) of the ultrasound probe, and the right side of the image data represents data captured by the side of the probe that is to a right of the sweeping direction of the ultrasound probe.

For the purposes of Fig. 7, when the illustrated orientation 720, 721 points upwards (e.g., orientation 720), this indicates that the predicted orientation of the representation of the portion of the fetus is that the fetus lies generally in the first direction (i.e., the head of the fetus is angled towards the last or the later slice in the sequence).

Similarly, when the illustrated orientation 720, 721 points downwards (e.g., orientation 721), this indicates that the predicted orientation of the representation of the portion of the fetus is that the fetus lies generally in the second direction toward (i.e., the head of the fetus is angled towards the first or the earlier slice in the sequence).

Fig. 7 thereby illustrates the relationship between the location of the representation of the spine 710 in the image slice, the predicted orientation 720 and the laterality (L, R) of the fetus. For completeness, Fig. 7 also illustrates, for each image slice, a direction 730 pointing away from the spine (e.g., and along a bisecting line for the image slice).

By way of first example 710, if the predicted orientation 720 of the representation of the portion of the fetus is in a direction that the head of the fetus is angled towards the last image slice of the ultrasound image data, or in other words, towards the forward direction of the sweeping (i.e., the first direction), then, for the target image, the identified portion of the target image that lies clockwise with respect to forward direction for the target slice represent a left side of the fetus, with the identified portion of the target image that lies anticlockwise with respect to the forward direction for the target slice represent a right side of the fetus.

By way of second example 702, if the predicted orientation 721 of the representation of the portion of the fetus is in a direction that the head of the fetus is angled towards the first image slice of the ultrasound image data or, in other words, towards the backward direction of the sweeping (i.e., the second direction), then, for the target image, the identified portion of the target image that lies anticlockwise with respect to the forward direction for the target slice represent a left side of the fetus, with the identified portion of the target image that lies clockwise with respect to the forward direction for the target slice represent a right side of the fetus.

The preceding examples make use of one or more target image slices of the ultrasound image data. The one or more target image slices may, for instance comprise (only): each image slice of the ultrasound image data; each image slice of the ultrasound image data that contains a representation of the spine; each image slice of the ultrasound image data that contains a representation of the heart of the fetus or each image slice that is currently being displayed, or has been selected for display, at an output user interface.

However, these examples are not exhaustive, and the skilled person would readily understand other options and variants for defining the at least one target slice of the ultrasound image data.

In some examples, the method 500 further comprises, for each target image slice, a step 540 of annotating the portions of the target image slice using the identifying information to identify which portion represents the right side and which portion represents the left side. A simple approach for annotating the portions may be to simply label the left portion with an "L" and the right side portion with an "R". Thus, labels (e.g., "L" and "R" labels) may be positioned on each image slice.

In particular, step 540 may comprise annotating the portions of the image slice comprises annotating the image slice outside the (identified) bounds of the representation of the fetus.

The bounds of the representation of the fetus may have been previously identified (e.g., in some versions of step 530) or may be identified when performing step 540. In this way, the method may comprise a step of, for each target image slice, processing the target image slice to identify the bounds of the representation of the fetus within the identified image slice.

The computer-implemented method 500 may further comprise a step 550 of controlling a display to provide a visual representation of at least one target image slice including the annotations of the left side and the right side of the fetus.

Approaches for controlling a display to provide a visual representation of an element, such as ultrasound image data and/or information about a predicted orientation, are well known and established in the art.

The computer-implemented method 500 may further comprise a step 560 of processing the identifying information, using a situs detection algorithm, to identify the position of each representation of one or more anatomical elements and/or anatomical organs within one or more of the at least one target image slices.

Although not illustrated for the sake of conciseness, the method 500 may further comprise the additional steps set out in Fig. 4 and the accompanying description, e.g., to determine and optionally display an absolute orientation.

The skilled person would be readily capable of developing a processing system for carrying out any herein described method. Thus, each step of the flow chart may represent a different action performed by a processing system, and may be performed by a respective module of the processing system.

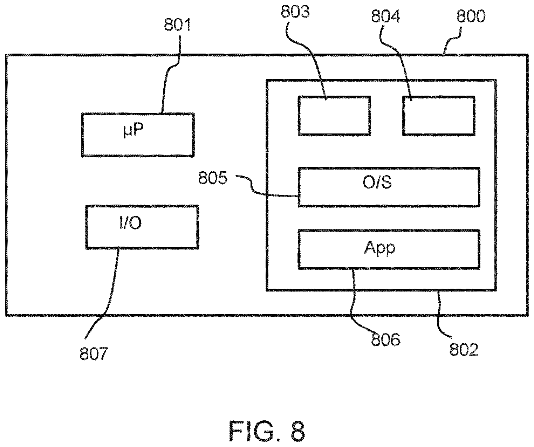

Fig. 8 illustrates an example of a processing system 800 within which one or more parts of an embodiment may be employed. Various operations discussed above may utilize the capabilities of the processing system. For example, one or more parts of a proposed embodiment may be incorporated in any element, module, application, and/or component discussed herein. In this regard, it is to be understood that system functional blocks can run on a single processing system or may be distributed over several processing systems and locations (e.g., connected via internet), such as a cloud-based computing infrastructure.

The processing system 800 includes, but is not limited to, PCs, workstations, laptops, PDAs, palm devices, servers, storages, and the like. Generally, in terms of hardware architecture, the processing system 800 may include one or more processors 810, memory 820 and one or more I/O devices 830 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 810 is a hardware device for executing software that can be stored in the memory 820. The processor 810 can be virtually any custom made or commercially available processor, a central processing unit (CPU), a digital signal processor (DSP), or an auxiliary processor among several processors associated with the processing system 800, and the processor 810 may be a semiconductor based microprocessor (in the form of a microchip) or a microprocessor.

The memory 820 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 820 may incorporate electronic, magnetic, optical, and/or other types of storage media. Note that the memory 820 can have a distributed architecture, where various components are situated remote from one another, but can be accessed by the processor 810.

The software in the memory 820 may include one or more separate programs, each of which comprises an ordered listing of executable instructions for implementing logical functions. The software in the memory 820 includes a suitable operating system (O/S) 850, compiler 860, source code 870, and one or more applications 880 in accordance with exemplary embodiments. As illustrated, the application 880 comprises numerous functional components for implementing the features and operations of the exemplary embodiments. The application 880 of the processing system 800 may represent various applications, computational units, logic, functional units, processes, operations, virtual entities, and/or modules in accordance with exemplary embodiments, but the application 880 is not meant to be a limitation.

The operating system 850 controls the execution of other computer programs, and provides scheduling, input-output control, file and data management, memory management, and communication control and related services. It is contemplated by the inventors that the application 880 for implementing exemplary embodiments may be applicable on all commercially available operating systems.

Application 880 may be a source program, executable program (object code), script, or any other entity comprising a set of instructions to be performed. When a source program, then the program is usually translated via a compiler (such as the compiler 860), assembler, interpreter, or the like, which may or may not be included within the memory 820, so as to operate properly in connection with the O/S 850. Furthermore, the application 880 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 830 may include input devices such as, for example but not limited to, a mouse, keyboard, scanner, microphone, camera, etc. Furthermore, the I/O devices 830 may also include output devices, for example but not limited to a printer, display, etc. Finally, the I/O devices 830 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

If the processing system 800 is a PC, workstation, intelligent device or the like, the software in the memory 820 may further include a basic input output system (BIOS) (omitted for simplicity). The BIOS is a set of essential software routines that initialize and test hardware at start-up, start the O/S 850, and support the transfer of data among the hardware devices. The BIOS is stored in some type of read-only-memory, such as ROM, PROM, EPROM, EEPROM or the like, so that the BIOS can be executed when the processing system 800 is activated.

When the processing system 800 is in operation, the processor 810 is configured to execute software stored within the memory 820, to communicate data to and from the memory 820, and to generally control operations of the processing system 800 pursuant to the software. The application 880 and the O/S 850 are read, in whole or in part, by the processor 810, perhaps buffered within the processor 810, and then executed.

When the application 880 is implemented in software it should be noted that the application 880 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 880 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The proposed method(s), device(s) and/or system(s) may be implemented in hardware or software, or a mixture of both (for example, as firmware running on a hardware device). To the extent that an embodiment is implemented partly or wholly in software, the functional steps illustrated in the process flow diagrams may be performed by suitably programmed physical computing devices, such as one or more central processing units (CPUs) or graphics processing units (GPUs). Each process - and its individual component steps as illustrated in the flow diagrams - may be performed by the same or different computing devices. According to embodiments, a computer-readable storage medium stores a computer program comprising computer program code configured to cause one or more physical computing devices to carry out a control method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, some of the blocks shown in the block diagrams may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

Variations to the disclosed embodiments can be understood and effected by those skilled in the art in practicing the claimed invention, from a study of the drawings, the disclosure and the appended claims. The mere fact that certain measures are recited in mutually different dependent claims does not indicate that a combination of these measures cannot be used to advantage.

In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

A single processor or other unit may fulfill the functions of several items recited in the claims. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. A computer-implemented method for processing ultrasound image data of a fetus, the computer-implemented method comprising:
receiving ultrasound image data containing a 3D representation of a portion of the fetus, wherein the ultrasound image data is formed from a temporal sequence of image slices, each image slice being captured at a different point in time;
processing the ultrasound image data to identify, for each of a plurality of predetermined views of the fetus, a respective image slice containing a representation of said predetermined view; and
predicting an orientation of the representation of the portion of the fetus within the ultrasound image data responsive to an order in which the identified image slices are located within the temporal sequence of image slices.

2. The computer-implemented method of claim 1, wherein the step of predicting the orientation comprises:
determining an order in which the identified image slices appear in the temporal sequence of image slices; and
mapping the determined order to an orientation of the representation of the portion of the fetus.

3. The computer-implemented method of any of claims 1 or 2, wherein the orientation of the representation of the portion of the fetus represents an overall orientation of the fetus.

4. The computer-implemented method of any one of claims 1 to 3, wherein the plurality of predetermined views includes at least one view of the heart of the fetus.

5. The computer-implemented method of claim 4, wherein the plurality of predetermined views includes two or more different views of the heart of the fetus.

6. The computer-implemented method of any of claims 1 to 5, further comprising:
processing the ultrasound image data to identify, as a spine location, a location of a representation of the spine of the fetus in the ultrasound image data; and
processing the identified spine location and the predicted orientation of the representation of the portion of the fetus to identify a left side and a right side of the fetus in the ultrasound image data.

7. The computer-implemented method of claim 6, further comprising:
for each of at least one target image slice of the ultrasound image data, processing the target image slice to identify a location of a bisecting line that is predicted to bisect the target image slice into two portions, each portion representing a different half of the representation of the portion of the fetus; and
generating identifying information, the identifying information identifying which portion of each target image slice represents the left side of the fetus and which portion represents the right side of the fetus, using the identified spine location and the predicted orientation of the representation of the portion of the fetus.

8. The computer-implemented method of claim 7, further comprising, for each target image slice, annotating the portions of the target image slice using the identifying information to indicate which portion represents the right side and which portion represents the left side.

9. The computer-implemented method of claim 8, further comprising, for each target image slice, processing the target image slice to identify the bounds of the representation of the fetus within the target image slice, wherein
annotating the portions of the target image slice comprises annotating the target image slice outside the identified bounds of the representation of the fetus.

10. The computer-implemented method of claim 8 or 9, further comprising controlling a display to provide a visual representation of at least one target image slice including the annotations of the left side and the right side of the fetus.

11. The computer-implemented method of any one of claims 7 to 10, further comprising processing the identifying information using a situs detection algorithm to identify the position of each representation of one or more anatomical elements and/or anatomical organs within one or more of the at least one target image slices.

12. The computer-implemented method of any one of claims 1 to 11, further comprising controlling a display to provide a visual representation of the ultrasound image data and a visual representation of the predicted orientation.

13. A computer program product comprising computer program code means which, when executed on a computing device having a processing system, cause the processing system to perform all of the steps of the method according to any one of claims 1 to 12.

14. A processing system for processing ultrasound image data of a fetus, the processing system being configured to:
receive ultrasound image data containing a 3D representation of a portion of the fetus, wherein the ultrasound image data is formed from a temporal sequence of image slices, each image slice being captured at a different point in time;
process the ultrasound image data to identify, for each of a plurality of predetermined views of the fetus, a respective image slice containing a representation of said predetermined view; and
predict an orientation of the representation of the portion of the fetus within the ultrasound image data responsive to an order in which the identified image slices are located within the temporal sequence of image slices.

15. A system comprising:
the processing system of claim 14; and
an ultrasound imaging system configured to generate the ultrasound image data.
